Europäisches Patentamt

(19) **European Patent Office**

Office européen des brevets

(11) Publication number: **0 112 849**
**B1**

(12) **EUROPEAN PATENT SPECIFICATION**

(45) Date of publication of patent specification: **16.09.87**

(51) Int. Cl.⁴: **C 12 N 15/00**, C 12 N 9/52 //
C12N9/60, C12R1/19

(21) Application number: **83901829.8**

(22) Date of filing: **07.06.83**

(86) International application number:
**PCT/GB83/00152**

(87) International publication number:
**WO 83/04418 22.12.83 Gazette 83/29**

(54) **A PROCESS FOR THE PREPARATION OF CHYMOSIN.**

(30) Priority: **07.06.82 CA 404600**
**25.03.83 GB 8308234**

(43) Date of publication of application:
**11.07.84 Bulletin 84/28**

(45) Publication of the grant of the patent:
**16.09.87 Bulletin 87/38**

(84) Designated Contracting States:
**AT BE CH DE FR LI LU NL SE**

(56) References cited:
**EP-A-0 035 781**
**EP-A-0 068 691**

**Chemical Abstracts, vol. 87, no. 19, 7
November 1977, Columbus, Ohio (US);
D.M.MULVIHILL et al.: "Selective denaturation
of milk coagulants in 5 M-urea", p. 216, abstract
147755e, J. Dairy Res. 1977, 44(2), 319-24
H.R. Mahler and E.H. Cordes: "Biological
Chemistry", 2nd edition, Harper & Row, 1971,
New York (US); pp. 177-183**

(73) Proprietor: **CELLTECH LIMITED**
**244-250 Bath Road**
**Slough Berkshire SL1 4DY (GB)**

(72) Inventor: **CAREY, Norman Henry**
**Russells Close High Street**
**Chinnor Oxfordshire (GB)**
Inventor: **DOEL, Michael Terence**
**Woodbury Wycombe Road**
**Studley Green Buckinghamshire (GB)**
Inventor: **HARRIS, Timothy John Roy**
**54 Red Rose Binfield**
**Bracknell Berkshire (GB)**
Inventor: **LOWE, Peter Anthony**
**9 Melrose Avenue**
**Reading Berkshire (GB)**
Inventor: **EMTAGE, John Spencer**
**12 Benjamin House Amersham Hill**
**High Wycombe Buckinghamshire (GB)**

(74) Representative: **Votier, Sidney David et al
CARPMAELS & RANSFORD 43, Bloomsbury
Square
London WC1A 2RA (GB)**

Courier Press, Leamington Spa, England.

## Description

### Field of the Invention

This invention relates to the field of protein production using recombinant DNA biotechnology. In particular it relates to a process for preparing chymosin from an insoluble form of a chymosin precursor produced by a host organism transformed with a vector including a gene coding for the chymosin percursor.

### Background of the Invention

There are now numerous examples of commercially valuable proteins which may be produced in large quantities by culturing a host organism capable of expressing heterologous genetic material. Once a protein has been produced by a host organism it is usually necessary to treat the host organism in some way, in order to obtain the desired protein in a free form. In some cases, such as in the production of the interferon in E. coli a lysis or permeabilisation treatment alone may be sufficient to afford satisfactory yields. However, some proteins are produced within a host organism in the form of insoluble protein aggregates which are not susceptible to extraction by lysis or permeabilisation treatment alone. It has been reported for instance that a human insulin fusion protein produced in E. coli forms insoluble protein aggregates (see D. C. Williams et al. Science Vol. 215 pages 687—689). In the normal biologically active form (hereinafter referred to as the native form) a protein exists as a chain of amino acids linked by peptide bonds. The chain is folded into a thermodynamically preferred three dimensional structure, the conformation of which is maintained by relatively weak interatomic forces such as hydrogen bonding, hydrophobic interactions and charge interactions. A number of S—S covalent bonds may form intramolecular bridges in the polypeptide chain. The insoluble proteins produced by certain host organisms do not exhibit the functional activity of their natural counterparts and are therefore in general of little use as commercial products. The lack of functional activity may be due to a number of factors but it is likely that such proteins produced by transformed host organisms are formed in a conformation which differs from that of their native states. The altered three dimensional structure of such proteins not only leads to insolubility but also diminishes or abolishes the biological activity of the protein. It is not possible to predict whether a given protein expressed by a given host organism will be soluble or insoluble.

In our copending published British patent application GB2100737A we describe a process for the production of the proteolytic enzyme chymosin. The process involves cleaving one of the chymosin precursor polypeptides; preprochymosin, methionine-prochymosin or methionine-chymosin, which may be expressed from a host organism which has been transformed with a vector including a gene coding for the relevant protein. The process for preparing a host organism transformed with a vector carrying a suitable gene is described in detail in the specification of our published British patent application GB2100737A and the teachings thereof are incorporated herein by reference.

In the course of our work relating to the process of preparing chymosin we discovered that the chymosin precursor proteins produced by various host organisms used were not produced in their native form. In particular the methionine-prochymosin produced by E. coli. is almost entirely produced as an insoluble aggregate and about 75% of the methionine-prochymosin produced in Saccharomyces cerevisiae is produced in an insoluble form.

In order to produce a chymosin precursor in a native form which may be cleaved to form active native chymosin the proteins produced by a host organism must be solubilised and converted into their native form before the standard techiques of protein purification and cleavage may be applied.

### Summary of the Present Invention

According to the present invention we provide a process for production of chymosin in which an insoluble form of a chymosin presursor is produced by a host organism transformed with a vector including a gene coding for the chymosin precursor wherein the insoluble form of the chymosin precursor is solubilised to produce the soluble native form of the chymosin precursor prior to cleaving the soluble native form of the chymosin precursor to produce chymosin.

Preferably the insoluble form of the chymosin precursor produced by the host organism is preprochymosin or a fusion protein including the amino acid sequence of preprochymosin, prochymosin or chymosin, for example, methionine-prochymosin or methionine-chymosin. Methionine-prochymosin is preferred as the chymosin precursor. The host organism may be a host organism or the progeny of a host organism which has been transformed (using the techniques described in our published British patent application GB2100737A) with a vector including heterologous genetic material coding for the chymosin precursor. The host organism may be a yeast, for example, Saccharomyces cerevisiae or a bacterium, for example, E. coli, B. subtilis, B. stearothermophilis, or Pseudomonas. Saccharomyces cerevisiae or E. coli are the preferred host organisms.

The expression system comprising E. coli transformed with a vector including a gene coding for the chymosin precursor, methionine-prochymosin, is especially preferred.

The term 'insoluble' as used herein means in a form which, under substantially neutral conditions (for example pH in the range 5.5 to 8.5), is substantially insoluble or is in insolubilised association with insoluble material produced on lysis of host organism cells. The insoluble product is either produced within the cells of the host organism in the form of insoluble relatively high molecular weight aggregates or may simply be associated with insoluble cell membrane material.

In order to produce the native form of the chymosin precursor it is necessary to alter the conformation of the insoluble product by the host organism. This may be done by denaturing the insoluble protein. Denaturing has the effect of abolishing the weak interatomic forces which maintain the protein in its three dimensional form causing the protein to unfold. The covalent bonds between adjacent atoms in the protein are left intact, including S—S bonds which maintain some of the three dimensional structure of the protein. The denatured state of a protein is less compact and is generally catalytically inactive. The denatured state is however usually soluble in the denaturing solution used. Removal of the denaturant from the solubilised proteins results in the refolding of the protein to produce the thermodynamically preferred native state of the protein. The renaturing is accompanied by the appearance of biological activity.

According to a preferred aspect of the invention the solubilisation involves reversibly denaturing the insoluble form of the chymosin precursor and subsequently allowing the chymosin precursor to renature, thereby producing the soluble native form of the chymosin precursor.

Preferably the insoluble form of the chymosin precursor is denatured in an aqueous solution comprising urea at a concentration of at least 7M and the chymosin precursor is renatured subsequently by reducing the concentration of urea in the solution below a concentration effective to denature the chymosin precursor, to produce a soluble native form of the chymosin precursor.

When the insoluble chymosin precursor is treated with urea the insoluble precursor is completely solubilised. The disulphide intramolecular bridges of the protein are however preserved and subsequently act as a nucleus for refolding. When the urea is removed, for example by dialysis, the protein returns to a thermodynamically stable conformation which, in the case of chymosin precursors, is a conformation capable of being converted to active chymosin by the methods described in published British patent application GB2100737A. The renatured proteins have the solubility characteristics of the native proteins.

In a further preferred aspect the insoluble form of the chymosin precursor is denatured in an aqueous solution comprising guanidine hydrochloride at a concentration of at least 6M and the chymosin precursor is renatured subsequently by reducing the concentration of guanidine hydrochloride in the solution below a concentration effective to denature the chymosin precursor, to produce a soluble native form of the chymosin precursor.

The physical effect of using guanidine hydrochloride is as discussed above for urea.

In a further preferred aspect the insoluble form of the chymosin precursor is denatured in an alkaline aqueous solution of between pH 9 and pH 11.5 and the chymosin precursor is renatured subsequently by reducing the pH of the solution below a pH effective to denature the chymosin precursor, to produce the soluble native form of the chymosin precursor. Preferably the alkaline aqueous solution is of pH between pH 10 and pH 11. Most preferred is an alkaline aqueous solution of pH from 10.5 to 10.9, preferably about 10.7.

Treatment of an insoluble chymosin precursor extract with an alkaline solution as described above does not result in complete solubilisation of the chymosin precursor. Since insoluble material such as cell debris is present at all times, a number of mass transfer effects are important. It has been found that multiple extractions with alkali are more efficient than a single extraction even when large extraction volumes are used. This also has the advantage of minimising the time for which the solubilised chymosin precursor is in contact with alkali. This is of importance since there is evidence that prochymosin slowly loses activity in alkaline solutions. Preferably, therefore, in this aspect of the invention the insoluble form of the chymosin precursor is present in conjunction with debris derived from the host organism which is insoluble in the aqueous solution and wherein one or more extractions of denatured chymosin precursor are performed. In view of the relativey low cost of the materials involved, the alkali solubilisation technique is attractive in terms of commercial exploitation.

The methods of solubilisation in a strong denaturant such as guanidine hydrochloride or urea and solubilisation using alkali, each solubilise significant percentages of the insoluble chymosin precursor which are found in extracts from host organisms. However, neither is quantitative in terms of recovery of native chymosin precursor. The reasons for this have not been clearly defined and are probably different for the two types of solubilisation. It appears that guanidine hydrochloride solubilises all the material present but only a portion is converted into proteins capable of activation to chymosin. Alkali treatment may not allow complete renaturation to form the native form of the chymosin precursor but in addition, does not solubilise all the insoluble form of the chymosin precursor. We have discovered that by combining the two methods a greatly enhanced recovery of chymosin precursor in its native state may be obtained.

According to a further preferred aspect of the present invention the insoluble form of the chymosin precursor is denatured in an aqueous solution, the resulting solution is diluted into 10 to 50 volumes of an alkaline aqueous solution of between pH 9 and pH 11.5 and the chymosin precursor is renatured by reducing the pH of the solution below a pH effective to denature the chymosin precursor, to produce the soluble native form of the chymosin precursor.

Preferably the solution containing the denatured chymosin precursor is diluted into an alkaline aqueous solution of pH between pH 10 and pH 11 and more preferably into an alkaline aqueous solution of pH from 10.5 to 10.9, preferably about 10.7.

The dilution introduces an element of physical separation between the denatured molecules, before renaturation is brought about, for example, by neutralisation of the alkaline denaturing solution. The dilution and resulting physical separation of the denatured molecules appears to assist their renaturation. The solubilisation process described immediately above leads to a recovery, in the case of methionine-prochymosin, of more than 30% compared to, for example, 10 to 20% for the multiple alkali extractions also described above.

Preferably, in the combined solubilisation process described above the insoluble form of the chymosin precursor is denatured in an aqueous solution comprising urea at a concentration of at least 7M or in a solution comprising guanidine hydrochloride at a concentration of at least 6M.

The present invention is preferably applied to the solubilisation of insoluble methionine-prochymosin produced by a host organism transformed with a vector including a gene coding for methionine-prochymosin. Preferably the host organism is *E. coli*.

A process for preparing host organisms coding for a number of chymosin precursors, including methionine-prochymosin, is described in detail in our published British patent application GB-2100737A. In addition published British patent application GB2100737A contains details for producing active chymosin from a chymosin precursor prepared in its native form by the solubilisation process of the present invention.

Some embodiments of the present invention are now described in detail by way of Examples.

Example 1

An experiment was conducted in which the solubilisation of insoluble methionine-prochymosin produced by *E. coli* cells transformed with vector pCT70 was achieved using urea or guanidine hydrochloride as denaturant. The preparation of the transformed *E. coli* cell line is described in detail in published British patent application GB2100737A.

Frozen *E. coli*/pCT70 cells grown under induced conditions were suspended in three times their weight of 0.05 M tris-HC1 pH8, 1 mM EDTA, 0.233 M NaCl, 10% glycerol (v/v) containing 130 µg/ml of lysozyme and the suspension was incubated at 4° for 20 minutes. Sodium deoxycholate was added to a final concentration of 0.05% and 10 µg of DNAase 1 (from bovine pancreas) was added per gram of *E. coli* starting material. The solution was incubated at 15°C for 30 minutes by which time the viscosity of the solution had decreased markedly. An equal volume of a solution containing 0.01 M tris pH 8, 0.1 mM EDTA, 5% glycerol was added and the extract centrifuged for 45 minutes at 4°C and 10000 × g. At this stage effectively all the methionine-prochymosin product was in the pellet fraction, presumably as a result of aggregation or binding to cellular debris. The pellet was washed in 50 volumes of 0.01 M tris. HCl, pH 8, 0.1 M NaCl, 1 mM EDTA at 4°. After further centrifugation as above the supernatant solution was discarded and the pellet rapidly solubilised at room temperature in a buffer containing either 8 M urea or 6 M guanidine hydrochloride, 0.05 M tris. HCl pH8, 1 mM EDTA and 0.1 M NaCl. It was then dialysed overnight at 4° against 200 volumes of 0.01 M tris. HCl pH8, 1 mM EDTA 0.1 M NaCl and 10% glycerol. A heavy precipitate formed (insoluble E. coli proteins) which was removed by centrifugation to leave a solution of native methionine-prochymosin protein. The soluble methionine-prochymosin was in a form which could be converted catalytically to active chymosin by acidification/neutralization, substantially as described in published British patent application GB2100737A.

Example 2

An experiment was conducted in which the solubilisation of insoluble methionine-prochymosin produced by *E. coli* cells transformed with vector pCT70 was achieved using alkaline denaturation. The preparation of the transformed *E. coli* cell line is described in detail in published British patent application GB2100737A.

Frozen *E. coli*/pCT70 cells grown under induced conditions were suspended in three times their own weight of 0.05 M Tris-HCl pH 8, 1 mM EDTA, 0.1 M NaCl, containing 23 µg/ml PMSF and 130 µg/ml of lysozyme and the suspension was incubated at 4°C for 20 minutes. Sodium deoxycholate was added to a final concentration of 0.05% and 10 µg of DNAase 1 (from bovine pancreas) was added per gram of *E. coli* starting material. The solution was incubated at 15°C for 30 minutes by which time the viscosity of the solution had decreased markedly. The extract, obtained as described above, was centrifuged for 45 minutes at 4°C and 10000 × g. At this stage effectively all the methionine-prochymosin product was in the pellet fraction in insolubilised form, presumably as a result of aggregation or binding to cellular debris. The pellet was washed in 3 volumes of 0.01 M tris-HCl pH 8, 0.1 M NaCl, 1 mM EDTA at 4°C. After further centrifugation, as above, the supernatant solution was discarded and the pellet resuspended in 3 volumes of alkali extraction buffer: 0.05 M K₂HPO₄, 1 mM EDTA, 0.1 M NaCl, pH 10.7 and the suspension adjusted to pH 10.7 with sodium hydroxide. The suspension was allowed to stand for at least 1 hour (and up to 16 hours) at 4°C, the pH of the supernatant adjusted to 8.0 by addition of concentrated HCl and centrifuged as above. Methionine-prochymosin, representing a substantial proportion of the methionine-prochymosin originally present in the pellet, was found to be present in the supernatant in a soluble form which could be converted to catalytically active chymosin by acidification/neutralisation activation treatment substantially as described in published British patent application GB2100737A.

We further noticed that re-extraction of the debris left after the first alkali extraction liberates an equivalent amount of prochymosin. Alkali extraction may be repeated to a total of 4—5 times with the liberation of approximately equivalent levels of prochymosin at each extraction.

Example 3

An experiment was conducted in which the solubilisation of methionine-prochymosin produced by E. coli cells transformed with vector pCT70 was acheived during denaturation with guanidine hydrochloride, followed by dilution into an alkaline solution. The preparation of the transformed cell line is described in detail in published British application GB2100737A.

E. coli/pCT70 cell debris containing insoluble methionine-prochymosin was prepared and washed as described in Example 1 above and the following manipulations were carried out at room temperature. The cell debris was dissolved in 3—5 volumes of buffer to final concentration of 6M guanidine HCl/0.05 M Tris pH8, 1mM EDTA, 0.1m NaCl and allowed to stand for 30 mins. — 2 hrs. The mixture was diluted to 10—50 volumes of the above buffer at pH 10.7 lacking guanidine HCl. Dilution was effected by slow addition of the sample to the stirred diluent over a period of 10—30 minutes. The diluted mixture was readjusted to pH 10.7 by the addition of 1 M NaOH and allowed to stand for 10 mins. — 2hrs. The pH was then adjusted to 8 by the addition of 1N HCl and the mixture allowed to stand for a further 30 minutes before centrifuging as above to remove precipitated proteins. The supernatant so produced contained soluble methionine-prochymosin which could be converted to catalytically active chymosin by acidification and neutralisation and purified as described in published British patent application GB2100737A. In a very similar experiment an 8M urea buffer was used in place of the 6M guanidine HCl buffer described above. The results were as described above.

**Claims**

1. A process for the production of chymosin in which an insoluble form of a chymosin precursor is produced by a host organism transformed with a vector including a gene coding for the chymosin precursor, wherein the insoluble form of the chymosin precursor is solubilised to produce a soluble native form of the chymosin precursor prior to cleaving the soluble native form of the chymosin precursor to produce chymosin.

2. A process according to claim 1 wherein the solubilisation involves reversibly denaturing the insoluble form of the chymosin precursor and subsequently allowing the chymosin precursor to renature, thereby producing the soluble native form of the chymosin precursor.

3. A process according to claim 2 wherein the insoluble form of the chymosin precursor is denatured in an aqueous solution comprising urea at a concentration of at least 7M and the chymosin percursor is renatured subsequently by reducing the concentration of urea in the solution below a concentration effective to denature the chymosin precursor to produce the soluble native form of the chymosin precursor.

4. A process according to claim 2 wherein the insoluble form of the chymosin precursor is denatured in an aqueous solution comprising guanidine hydrochloride at a concentration of at least 6M and the chymosin precursor is renatured subsequently by reducing the concentration of guanidine hydrochloride in the solution below a concentration effective to denature the chymosin precursor, to produce a soluble native form of the chymosin precursor.

5. A process according to claim 2 wherein the insoluble form of the chymosin precursor is denatured in an alkaline aqueous solution of between pH 9 and pH 11.5 and the chymosin precursor is denatured subsequently by reducing the pH of the solution below a pH effective to denature the chymosin precursor, to produce the soluble native form of the chymosin precursor.

6. A process according to claim 5 wherein the insoluble form of the chymosin precursor is present in conjunction with debris derived from the host organism which is insoluble in the aqueous solution and wherein one or more extractions of denatured chymosin precursor are performed.

7. A process according to claim 2 wherein the insoluble form of the chymosin precursor is denatured in an aqueous solution, the resulting solution is diluted into 10—50 volumes of an alkaline aqueous solution of between pH 9 and pH 11.5 and the chymosin precursor is renatured by reducing the pH of the solution below a pH effective to denature the chymosin precursor, to produce the soluble native form of the chymosin precursor.

8. A process according to claim 7 wherein the insoluble form of the chymosin precursor is denatured in an aqueous solution comprising urea at a concentration of at least 7M.

9. A process according to claim 7 wherein the insoluble form of the chymosin precursor is denatured in an aqueous solution comprising guanidine hydrochloride at a concentration of at least 6M.

10. A process according to any one of the preceding claims wherein the chymosin precursor is methionine-prochymosin.

11. A process according to any one of the preceding claims wherein the host organism is E. coli.

**Patentansprüche**

1. Verfahren zur Herstellung von Chymosin, in welchem eine unlösliche Form eines Chymosin-Precursors von einem Wirtsorganismus, welcher mit einem Vektor transformiert ist, der eine Gen-Codierung für den Chymosin-Precursor aufweist, produziert wird, wobei die unlösliche Form des Chymosin-Precursors solubilisiert wird, um eine lösliche native Form des Chymosin-Precursors zur produzieren, bevor eine Spaltung der löslichen nativen Form des Chymosin-Precursors zur Herstellung des Chymosins erfolgt.

2. Verfahren nach Anspruch 1, wobei die Solubilisierung ein reversibles Denaturieren der unlöslichen Form des Chymosin-Precursors und ein nachfolgendes Renaturieren-Lassen des Chymosin-Precursors einschließt, wobei die lösliche native Form des Chymosin-Precursors hergestellt wird.

3. Verfahren nach Anspruch 2, wobei die unlösliche Form des Chymosin-Precursors in einer wäßrigen Lösung, welche Harnstoff in einer Konzentration von zumindest 7 M enthält, denaturiert wird und danach der Chymosin-Precursor renaturiert wird, indem die Konzentration des Harnstoffs in der Lösung unter eine Konzentration, die zur Denaturierung des Chymosin-precursors wirksam ist, verringert wird, um eine lösliche native Form des Chymosin-Precursors herzustellen.

4. Verfahren nach Anspruch 2, wobei die unlösliche Form des Chymosin-Precursors in einer wäßrigen Lösung, welche Guanidin-Hydrochlorid in einer Konzentration von zumindest 6 M enthält, denaturiert wird, und danach der Chymosin-Precursor renaturiert wird, indem die Konzentration des Guanidin-Hydrochlorids in der Lösung unter eine Konzentration, welche zur Denaturierung des Chymosin-Precursors wirksam ist, verringert wird, um eine lösliche native Form des Chymosin-precursors herzustellen.

5. Verfahren nach Anspruch 2, wobei die unlösliche Form des Chymosin-Precursors in einer alkalischen wäßrigen Lösung mit einem pH-Wert zwischen 9 und 11,5 denaturiert wird und der Chymosin-Precursor danach durch Verringerung des pH-Wertes der Lösung unter einen pH-Wert, welcher zur Denaturierung des Chymosin-Precursors wirksam ist, renaturiert wird, um die lösliche native Form des Chymosin-precursors herzustellen.

6. Verfahren nach Anspruch 5, wobei die unlösliche Form des Chymosin-Precursors in Verbindung mit der von dem Wirtsorganismus stammenden Debris, welche in der wäßrigen Lösung unlöslich ist, vorliegt, und eine oder mehrere Extraktion(en) des denaturierten Chymosin-Precursors durchgeführt wird bzw. werden.

7. Verfahren nach Anspruch 2, wobei die unlösliche Form des Chymosin-Precursors in einer wäßrigen Lösung denaturiert wird, die dabei erhaltene Lösung auf das 10—50—Fache ihres Volumens 34 einer alkalischen wäßrigen Lösung mit einem pH-Wert zwischen 9 und 11,5 verdünnt wird, und der Chymosin-Precursor durch Verringerung des pH-Wertes der Lösung unter einen pH-Wert, welcher zur Denaturierung des Chymosin-Precursors wirksam ist, renaturiert wird, um die lösliche native Form des Chymosin-Precursors herzustellen.

8. Verfahren nach Anspruch 7, wobei die unlösliche Form des Chymosin-Precursors in einer wäßrigen Lösung, welche Harnstoff in einer Konzentration von zumindest 7 M aufweist, denaturiert wird.

9. Verfahren nach Anspruch 7, wobei die unlösliche Form des Chymosin-Precursors in einer wäßrigen Lösung, welche Guanidin-Hydrochlorid in einer Konzentration von zumindest 6 M aufweist, denaturiert wird.

10. Verfahren nach einem der vorhergehenden Ansprüche, wobei der Chymosin-Precursor Methionin-Prochymosin ist.

11. Verfahren nach einem der vorhergehenden Ansprüche, wobei der Wirtsorganismus E.coli ist.

**Revendications**

1. Procédé de production de chymosine, dans lequel une forme insoluble d'un précurseur de chymosine est produite par un organisme hôte transformé avec un vecteur comprenant un gène codant pour le précurseur de chymosine, procédé dans lequel la forme insoluble du précurseur de chymosine est solubilisée pour produire une forme native soluble du précurseur de chymosine avant le clivage de la forme native soluble du précurseur de chymosine pour produire la chymosine.

2. Procédé selon la revendication 1, dans lequel la solubilisation implique la dénaturation réversible de la forme insoluble du précurseur de chymosine puis de laisser le précurseur de chymosine revenir à l'état naturel en produisant ainsi la forme native soluble du précurseur de chymosine.

3. Procédé selon la revendication 2, dans lequel la forme insoluble du précurseur de chymosine est dénaturée dans une solution aqueuse comprenant de l'urée à une concentration d'au moins 7 M, et l'on fait ensuite revenir le précurseur de chymosine à son état naturel en diminuant la concentration de l'urée dans la solution pour l'amener au-dessous d'une concentration efficace pour dénaturer le précurseur de chymosine pour produire la forme native soluble du précurseur de chymosine.

4. Procédé selon la revendication 2, dans lequel la forme insoluble du précurseur de chymosine est dénaturée dans une solution aqueuse comprenant du chlorhydrate de guanidine présent en une concentration d'au moins 6 M, et l'on fait ensuite revenir le précurseur de chymosine à son état naturel en réduisant la concentration du chlorhydrate de guanidine dans la solution pour l'amener au dessous d'une concentration efficace pour dénaturer le précurseur de chymosine pour produire une forme native soluble du précurseur de chymosine.

5. Procédé selon la revendication 2, dans lequel la forme insoluble du précurseur de chymosine est dénaturée dans une solution alcaline aqueuse dont le pH est compris entre 9 et 11,5, et l'on fait ensuite revenir le précurseur de chymosine à son état naturel en diminuant le pH de la solution pour l'amener au dessous d'un pH efficace pour dénaturer le précurseur de chymosine afin de produire la forme native soluble du précurseur de chymosine.

6. Procédé selon la revendication 5, dans lequel la forme insoluble du précurseur de chymosine est présente de concert avec des débris provenant de l'organisme hôte qui est insoluble dans la solution aqueuse, et dans lequel on effectue une ou plusieurs extractions du précurseur de chymosine dénaturé.

7. Procédé selon la revendication 2, dans lequel la forme insoluble du précurseur de chymosine est denaturée. en solution aqueuse, la solution résultante est diluée dans 10 à 50 volumes d'une solution alcaline aqueuse dont le pH est compris entre 9 et 11,5, et l'on fait revenir le précurseur à son état naturel en réduisant le pH de la solution pour l'amener au dessous d'un pH efficace pour dénaturer le précurseur de chymosine pour produire la forme native soluble du précurseur de chymosine.

8. Procédé selon la revendication 7, dans lequel la forme insoluble du précurseur de chymosine est dénaturée dans une solution aqueuse comprenant de l'urée présente en une concentration d'au moins 7 M.

9. Procédé selon la revendication 7, dans lequel la forme insoluble du précurseur de chymosine est dénaturée dans une solution aqueuse comprenant du chlorhydrate de guanidine présent en une concentration d'au moins 6 M.

10. Procédé selon l'une quelconque des revendications précédentes, selon lequel le précurseur de chymosine est de la méthionine prochymosine.

11. Procédé selon l'une quelconque des revendications précédentes, dans lequel l'organisme hôte est *E. coli*.